# EUROPEAN PATENT APPLICATION

(11) **EP 3 777 650 A1**
(43) Date of publication of application: **17.02.2021**
(21) Application number: 18912007.4
(22) Date of filing: 28.03.2018
(51) Int. Cl.: A61B 1/313

(54) **ENDOLUMINAL SURGERY SYSTEM**

(71) Applicant: Mao, Zhangfan, Wuhan, Hubei 430010 (CN)
(72) Inventor: MAO, Hang, Wuhan, Hubei 430010 (CN); MAO, Zhangfan, Wuhan, Hubei 430010 (CN)
(74) Representative: Dr. Gassner & Partner mbB
(86) International application number: PCT/CN2018/080972
(87) International publication number: WO 2019/183862

(57) **Abstract**

Disclosed is an endoluminal surgery system, comprising an operating device (1), a connecting rod (2) and a handle (3), wherein the operating device (1) comprises an operating instrument (11) and a connecting part (12). During surgery, the operating device (1) is placed into a lumen (D) through an incision (B), a tip of the connecting rod (2) then enters the lumen (D) along a puncture point and is fixedly connected to the connecting part (12) of the operating device (1), the other end of the connecting rod (2) is fixedly connected, in vitro, to the handle (3), and the operating instrument (11) is then controlled and adjusted in vitro by the handle (3) to achieve a corresponding surgical operation. The endoluminal surgery system can reduce damage to a patient's body and can also more conveniently carry out, in vitro, a surgical operation, improving the success rate of surgery.

## Description

### TECHNICAL FIELD

The present invention relates to the field of surgical instruments, and particularly to an intracavitary surgery system.

### BACKGROUND

In conventional thoracoscopic or laparoscopic surgery, when the surgical instruments enter the body cavity for operation, it is necessary to first make an incision of 5mm or so on the body surface and insert a trocar to form a passage for entry or exit of the surgical instruments. When the surgery is finished, the trocar is removed and the incisions are stitched. At present, the extreme case of the endoscopic surgery is single-incision endoscopic surgery, that is, the endoscopy, the operating part and the tissue or organ pulling instrument are inserted into the body cavity through a same incision. However, due to the limitation of the size of the single incision, when multiple surgical instruments enter the cavity through the same incision, the operation becomes inconvenient and difficult, and the risk control ability is poor when the accidents occur, which is disadvantage to popularization. Therefore, although the single-incision surgery has only one incision, the advantage of the single-incision surgery compared with multi-incision surgery is small.

### BRIEF DESCRIPTION OF THE INVENTION

In view of this, the present disclosure provides an intracavitary surgery system, which can reduce the injury to the patients because the rod diameter of intracavitary surgical instruments is very small. The intracavitary surgery system can help retract the tissues or organs during operation through a tiny needle wound which need no suture after surgery.

According to embodiments of the present disclosure, there is provided an intracavitary surgery system.

The intracavitary surgery system includes: a needle type connecting rod; an operating device including an operating part and a connecting part; and a handle for fixing the connecting rod. The connecting part is configured to be fixedly connected with the tip of the connecting rod in the body cavity.

In one embodiment, the connecting part includes: a fixing groove for accommodating the tip of the connecting rod. The fixing groove is provided with a first limiting part for fixing the connecting rod

In one embodiment, the tip of the connecting rod is provided with a second limiting part matched with the first limiting part.

In one embodiment, the first limiting partis a buckle, and the second limiting part is a clamping groove.

In one embodiment, the tip of the connecting rod is configured to be fixedly connected with fixing groove by threads.

In one embodiment, the first limiting part includes at least one elastic ring.

In one embodiment, the second limiting part is provided with a clamping groove and a guide groove. The connecting rod is fixed with the elastic ring by the clamping groove, and is separated from the elastic ring by the guiding groove.

In one embodiment, the first limiting part is provided with a through-hole. The first limiting partis fixedly connected with the fixing groove by an elastic element.

In one embodiment, the through-hole is provided with a fixing buckle for fixing the connecting rod.

In one embodiment, the second limiting partis a clamping groove. In a first state, the through-hole of the first limiting part is coaxially disposed with the fixing groove) to make the connecting rod go through the through-hole. And in a second state, the buckle in the through-hole is engaged with the clamping groove to fix the connecting rod.

In one embodiment, the first limiting partis an elastic jaw.

In one embodiment, the second limiting part is a clamping groove, and the clamping groove is configured to be fixedly connected with the jaw to fix the connecting rod.

In one embodiment, a rubber block may be arranged at one end of the fixing groove close to the operating part to protect the tip of the connecting rod.

In one embodiment, the connecting part further includes: a sliding groove arranged opposite to the fixing groove, a slider arranged in the sliding groove, and a pull line with one end fixedly connected with the slider to drive the slider to move. The slider and the operating part are connected.

In one embodiment, the intracavitary surgery system further includes: a hook needle with a groove or a hook arranged at the end.

In one embodiment, the connecting part further includes: a connecting piece extends along a side of the sliding groove.

In one embodiment, the operating partis a retractor clamp. The retractor clamp is connected with the connecting piece.

In one embodiment, the retractor clamp includes a first clamping arm and a second clamping arm. The first clamping arm and the second clamping arm are rotatably connected by a first hinge shaft.

In one embodiment, the first hinge shaft is provided with a first spring. Two ends of the first spring extend and are fixedly connected with the first clamping arm and the second clamping arm respectively.

In one embodiment, the diameter of the connecting rod is less than 5mm.

In one embodiment, the surface of the middle part of the connecting rod is provided with a frosted surface.

The present disclosure provides an intracavitary surgery system. The intracavitary surgery system includes: an operating device, a connecting rod and a handle. The operating device includes an operating part and a connecting part. During the surgery, the operating device can be placed in the body cavity through an incision, and the tip of the connecting rod enters the body cavity through the piercing point and is fixedly connected with the connecting part of the operating device. The other end, outside the body, of the connecting rod is connected with the handle. Then, the operating part can be controlled and adjusted through the handle outside the body to achieve the corresponding surgery procedures. The present disclosure reduces the injury to the patient's body, makes the surgical procedures outside the body more convenient, and improves the success rate of the operation.

### BRIEF DESCRIPTION OF THE DRAWINGS

The above and other objects, features, and advantages of the present disclosure will be more apparent from the following description of embodiments of the present disclosure with reference to the accompanying drawings, in which:
FIG. 1 is a schematic diagram of an intracavitary surgery system of the present disclosure;
FIG. 2 is a schematic diagram illustrating operations of an intracavitary surgery system of the present disclosure;
FIG. 3 is a schematic diagram of a retractor clamping an opening state according to the first embodiment of the present disclosure;
FIG. 4 is a schematic diagram of a retractor clamping a closing state according to the first embodiment of the present disclosure;
FIG. 5 is a first schematic diagram of a connecting part according to the first embodiment of the present disclosure;
FIG. 6 is a second schematic diagram of a connecting part according to the first embodiment of the present disclosure;
FIG. 7 is a third schematic diagram of a connecting part according to the first embodiment of the present disclosure;
FIG. 8 is a first schematic diagram of a hook needle according to the first embodiment of the present disclosure;
FIG. 9 is a second schematic diagram of the hook needle according to the first embodiment of the present disclosure;
FIG. 10 is a schematic diagram of a retractor clamping a closing state according to the second embodiment of the present disclosure;
FIG. 11 is a schematic diagram of a retractor clamping an opening state according to the second embodiment of the present disclosure;
FIG. 12 is a schematic diagram of a connecting part according to the third embodiment of the present disclosure;
FIG. 13 is a schematic diagram of a first limiting part in a natural state according to the third embodiment of the present disclosure;
FIG. 14 is a schematic diagram of a first limiting part in a pressed state according to the third embodiment of the present disclosure;
FIG. 15 is a first schematic diagram of an intracavitary surgery system according to the fourth embodiment of the present disclosure;
FIG. 16 is a second schematic diagram of an intracavitary surgery system according to the fourth embodiment of the present disclosure.

### DETAILED DESCRIPTION

The present disclosure is described below based on embodiments, but the present disclosure is not limited to these embodiments. In the following detailed description of the present disclosure, some specific details are described in detail. To those skilled in the art, the present invention can be fully understood without the description of these details. In order to avoid obscuring the essence of the present disclosure, well-known methods, procedures, processes, components, and circuits have not been described in detail.

In addition, it will be understood by those skilled in the art that the drawings provided herein are for illustrative purpose and are not necessarily drawn to scale.

Unless the context clearly requires otherwise, the words "comprising", "including" and the like in the entire specification and claims should be construed as the meaning of inclusion rather than the meaning of exclusion or exhaustion, that is, the meaning of "including but not limited to".

In the description of the present disclosure, it should be understood that the terms "first", "second" and the like are for illustrative purposes only and should not to be construed as indicating or implying relative importance. In addition, in the description of the present disclosure, the meaning of "a plurality of' means two or more, unless otherwise specified.

Unless otherwise expressly specified or defined, terms "mount", "couple", "connect" and "fix" should be understood in a broad sense, and for example, a connection may be a fixed connection, or a detachable connection, or an integrated connection; a connection may be a mechanical connection or an electric connection; a connection may be a direct connection, or an indirect connection via an intermediate medium, or may be an internal communication between two elements or an interaction between two elements. Unless otherwise expressly specified, the specific meanings of the above-mentioned terms as used herein could be understood by those skilled in the art according to specific situations.

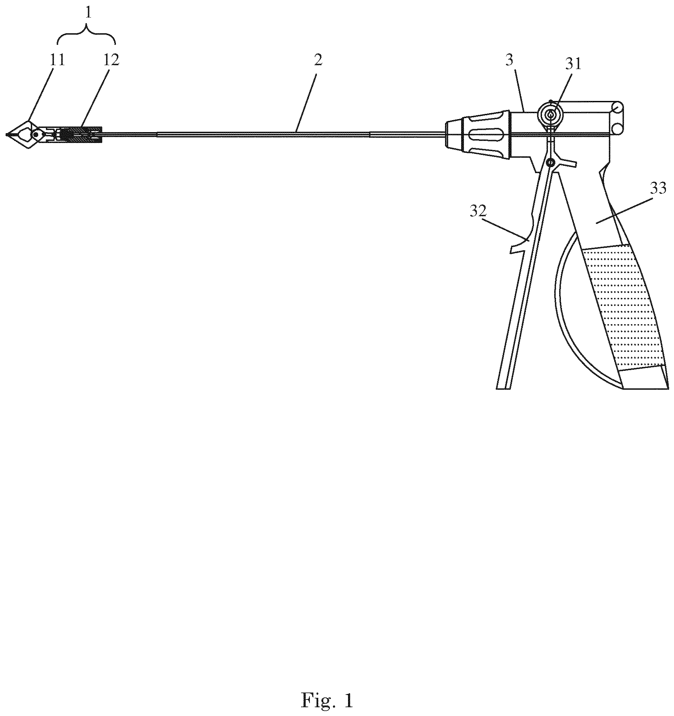

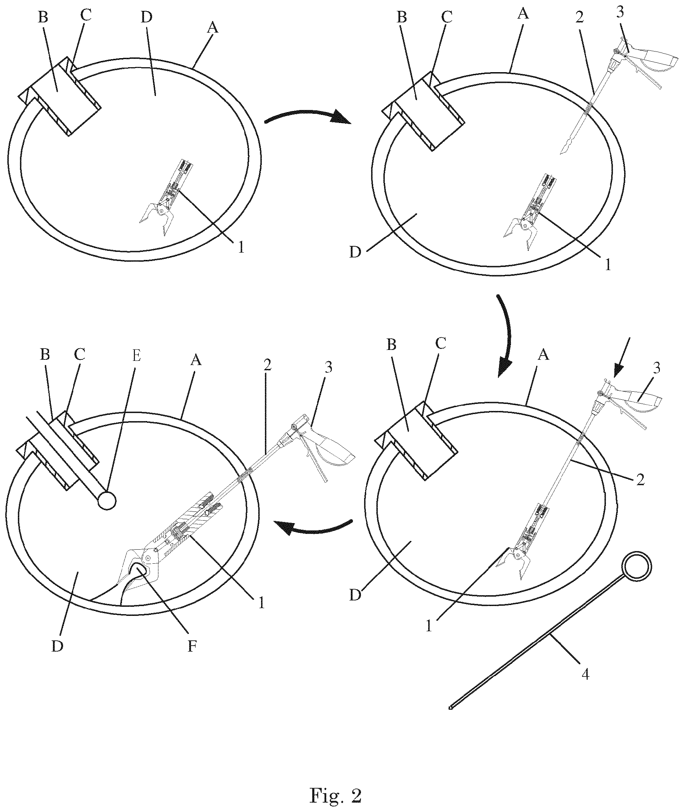

FIG. 1 is a schematic diagram of an intracavitary surgery system of the present disclosure. The intracavitary surgery system includes an operating device, a connecting rod 2 and a handle 3. The connecting rod 2 may be a solid needle or a hollow needle. If the connecting part 12 is provided with a pull line 124, the connecting rod 2 is a hollow needle. The operating device 1 is arranged in the thoracic or abdominal cavity for performing the corresponding operation on a lesion of the patient. Referring to FIG. 2, during the surgery, the surgeon makes an incision B on the patient's body surface A according to needs, and then inserts a trocar C corresponding to the size of the incision B for forming a passage for entry or exit of the surgical instruments. The surgeon places the operating device 1 through the trocar C into the body cavity D. The hollow needle 2 is fixedly connected with the handle 3, and the tip of the hollow needle 2 is pierced into the patient's body cavity D through another position on the patient's body surface A. The hollow needle 2 is fixedly connected with the operating device 1, and then, other surgical instrument such as an endoscope is placed in the body cavity D through the trocar C. The surgeon can control the operating device 1 through the handle 3 outside the body and perform the corresponding operation. For example, after clamping the organ F, the operation is performed by controlling the handle 3 for exposing the needed position. Preferably, the handle 3 can control the operating device 1 via the pull line 124, or the operating part 1 can be controlled by the handle 3 in a non-line manner via its own structure. When the handle 3 controls the operating device 1 by the pull line 124 and the hollow needle 2 is fixedly connected with the operating device 1, the hook needle 4 is inserted into the body cavity D through the end of the hollow needle 2 connected with the handle 3 in the direction of the straight arrow as shown in FIG. 2. The pull line 124 is connected with the handle 3, so that the pull line 124 fixed to the operating device 1 can be took out of the body cavity and fixedly connected with the handle 3.

If necessary, multiple operating devices 1 can be placed in the body cavity to clamp different parts of the tissue so as to better expose the operation part. The intracavitary surgery system of the embodiments of present disclosure can help avoid the mutual interference between multiple surgical instruments which enter the body cavity through the same incision during surgery, where the mutual interference affects the flexibility of surgical operation such as anatomy or separation, and also can reduce the compression and injury around the incision.

### First embodiment:

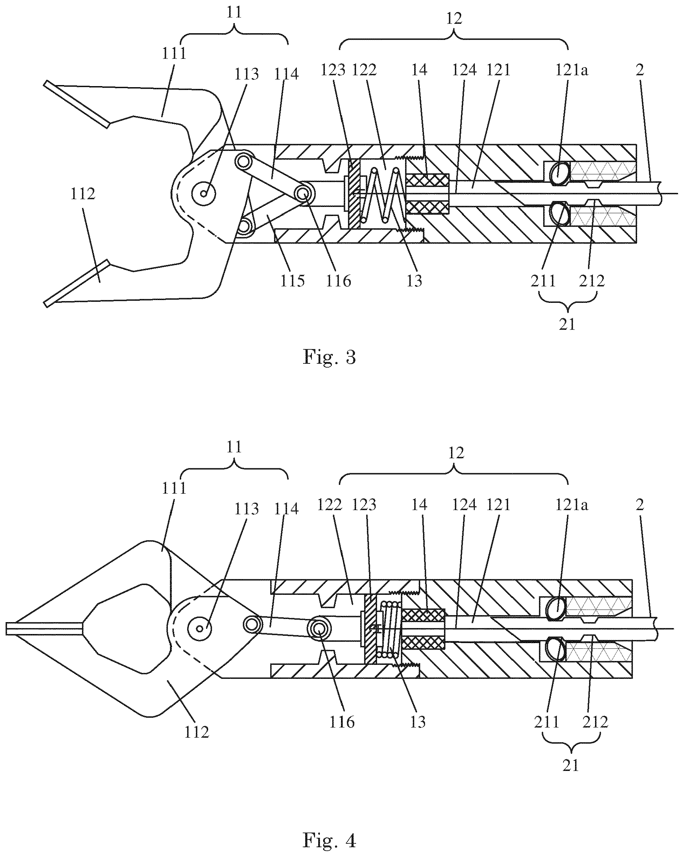

Referring to FIG. 3 and FIG. 4, in the present embodiment, the operating device 1 includes an operating part 11 and a connecting part 12. The operating part 11 may be a retractor clamp, a pair of medical scissors or other surgical instrument suitable for an endoscopic surgery. The connecting part 12 includes a fixing groove 121, a sliding groove 122, a slider 123 and a pull line 124. A first limiting part 121a for fixing the connecting rod 2 is arranged in the fixing groove 121. In this embodiment of the present disclosure, the connecting rod 2 is a hollow needle. The tip of the hollow needle 2 is provided with a second limiting part 21 which is matched with the first limiting part 121a. When the tip of the hollow needle 2 enters the fixing groove 121, the hollow needle 2 and the connecting part 12 are fixedly connected through the first limiting part 121a and the second limiting part 21. The connecting part 12 can be made of materials with high strength and hardness such as alloy steel, which can improve the strength of the operating device 1, and can also be used for making small size structures to meet usage requirements.

In this embodiment of the present disclosure, the first limiting part 121a may include at least one elastic ring, which is embedded in the fixing groove 121. The inner diameter of the elastic ring in the natural state is smaller than the outer diameter of the hollow needle 2, where the natural state refers to the state in which the elastic ring is not subjected to any external force. The second limiting part 21 may include a clamping groove 211 and a guide groove 212. The clamping groove 211 and the guide groove 212 are matched with the elastic ring. The clamping groove 211 and the guide groove 212 may be placed successively from the tip of the hollow needle 2 to a fixed end of the connecting rod 2. The clamping groove 211 is smaller than the guide groove 212. When the tip of the hollow needle 2 enters the fixing groove 121, the elastic ring is pressed into the fixing groove 121. Due to the small accommodation space of the clamping groove 211, the elastic ring forms an included angle with the insertion direction of the hollow needle 2. At this time, the pulling-out direction of the hollow needle 2 is different from the included angle of the elastic ring, so a resistance is relatively large, and it is difficult to pull out the hollow needle 2. When it needs to separate the hollow needle 2 from the connecting part 12 after the surgery is finished, the surgeon may insert the hollow needle 2 into the fixing groove 121 further. Since the insertion direction of the hollow needle 2 is the same as the included angle of the elastic ring, the resistance is relatively small, and the elastic ring can enter the guide groove 212 easily. Because the space of the guide groove 212 is larger, the elastic ring returns to its natural state. When the hollow needle 2 is pulled out, the elastic ring is compressed into an angle in the same direction as the pulling-out direction. Since the resistance is relatively small, it is easy to pull out the hollow needle 2 from the fixing groove 121 to realize the separation.

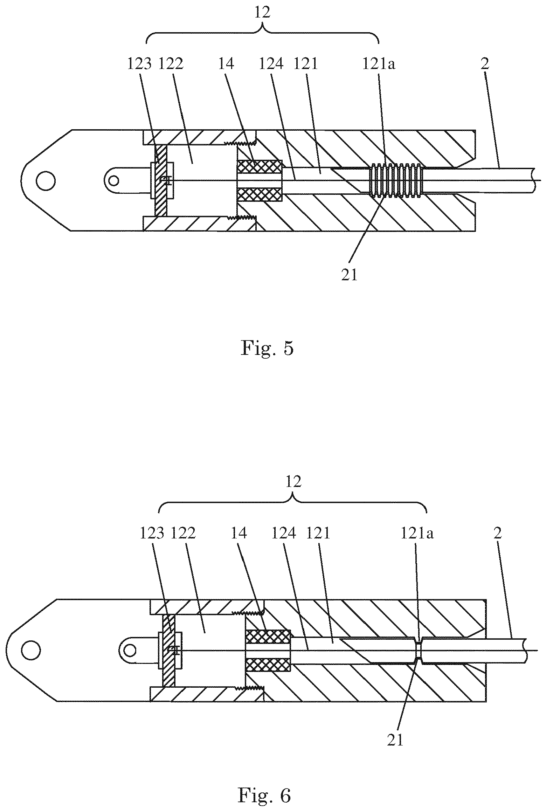

As shown in FIG. 5, in another implementation, the first limiting part 121a may be set to an internal thread and the second limiting part 21 of the hollow needle 2 may be set to an external thread matched with the internal thread. After the hollow needle 2 enters the body cavity through the piercing point, the hollow needle 2can be fixedly connected to the connecting part 12 by the threads.

As shown in FIG. 6, in another implementation, the first limiting part 121a may be set to an elastic buckle and the second limiting part 21 of the hollow needle 2 may be set to a clamping groove matched with the elastic buckle. After the hollow needle 2 enters the body cavity through the piercing point, the hollow needle 2 and the connecting part can be fixedly connected through the elastic buckle and the clamping groove.

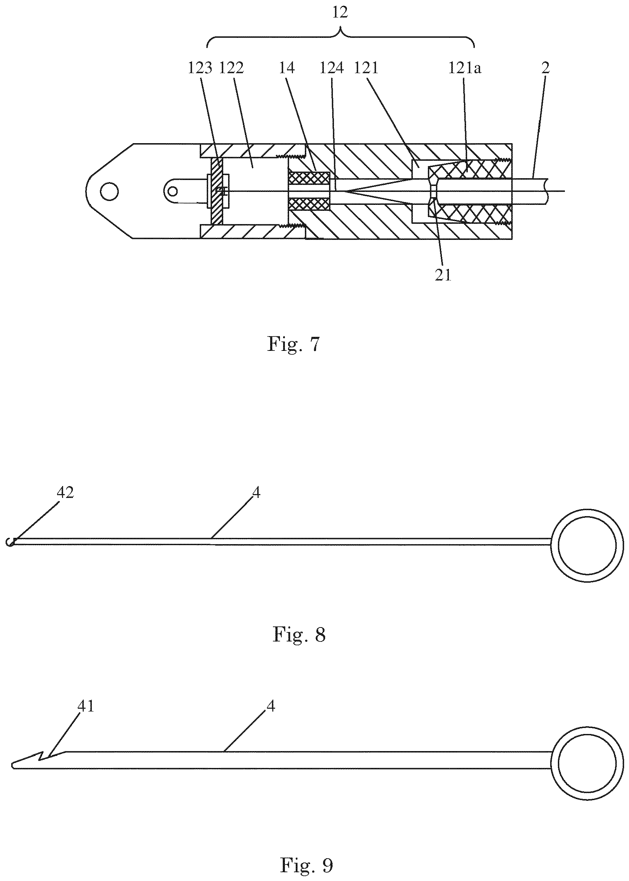

As shown in FIG. 7, in another implementation, the first limiting part 121a may be set to an elastic jaw, which is in the fixing groove 121 and fixed to the end of the fixing groove 121. The second limiting part may be a clamping groove. When the hollow needle 2 enters the body cavity through the piercing point, the elastic jaw moves along the tip of the hollow needle 2 based on its elasticity. When the elastic jaw reaching the clamping grooves, the accommodating space becomes larger, so the elastic jaw return to the natural state (the state in which they are not subjected to any external force) and be fixedly connected with the clamping groove.

The sliding groove 122 may be arranged on the side of the connecting part 12 opposite to the fixing groove 121, and the slider 123 may be placed in the sliding groove 122. One end of the slider 123 is connected with the operating part 11, and the operating part 11 is driven by the movement of the slider 123 in the sliding groove 122. The other end of the slider 123 is detachably connected with the pull line 124 for driving the operating part 11. After the hollow needle 2 enters the body cavity through the piercing point and is fixedly connected with the operating device 1, the surgeon causes the pull line 124 to pass through the hollow needle 2 and then control the operating part 11 by controlling the pull line 124 outside the body cavity.

The intracavitary surgery system further includes a hook needle 4. As shown in FIG. 8 and FIG. 9, the hook needle 4 includes a groove 41 or a hook 42 disposed at an end of the hook needle 4. The diameter of the hook needle 4 is smaller than the inner diameter of the hollow needle 2. After the hollow needle 2 passes through the body surface A and enters the body cavity D, the hook needle 4 is sent from outside into the body cavity D through the hollow needle 2. The pull line 124 is placed with the groove 41 or the hook 42 of the hook needle 4 and extends out of the hollow needle 2. Then, the hollow needle 2 is fixedly connected with the operating device 1 and the pull line 124 is connected with the handle 3, so that the pull line 124 is controlled through the handle 3 to move, and the operating part 11 is pulled accordingly. In the present embodiment, the operating part 11 is a retractor clamp. The surgeon can choose a specific retractor clamp according to the target tissues, such as a lung clamp, a stomach clamp, an intestinal clamp, a liver clamp, a tendon clamp, a vascular clamp, and the like. The retractor clamp may include a first clamping arm 111 and a second clamping arm 112. The first clamping arm 111 and the second clamping arm 112 may be rotatably connected through a first hinge shaft 113. The retractor clamp may be also connected with the connecting part 12 through the first hinge shaft 113. The inner surface of the first clamping arm 111 and the inner surface of the second clamping arm 112 may be provided with antiskid strips to prevent the tissues or organs from slipping out of the retractor clamp when the retractor clamp is in the close state. Each of the tail of the first clamping arm 111 and the tail of the second clamping arm 112 is provided with a mounting hole, and the two mounting holes are fixed with the connecting rod 114 and the connecting rod 115 respectively. The other ends of the connecting rod 114 and the connecting rod 115 are rotatably connected through a second hinge shaft 116. In the natural state (no external force is applied), an included angle between the connecting arms 114 and the connecting arms 115, and the retractor clamp is opened. When the pull line 124 pulls the slider 123 to the fixing groove 121, the included angle between the connecting arms 114 and the connecting arms 115 decreases gradually, which drives the first clamping arm 111 and the second clamping arm 112 to rotate relatively and clamp the target tissue or organ. Then the surgeon can move the target tissue or organ as needed to provide a good surgical field of view. To easily open the retractor clamp after the operation is complete, a reset spring 13 may be arranged in the space between the slider 123 and the fixing groove 121. When the pull line 124 is released, the slider 123 moves under the action of the elasticity of the reset spring 13, which drives the first clamping arm 111 and the second clamping arm 112 to rotate relatively so that the retractor clamp will open and return to the natural state.

Optionally, if it does not need to operate the operating part 11 temporarily during the surgery, the surgeon may use handle 3 and the hollow needle 2 from outside to pull the operating part 11 to a position near the body cavity, and a vascular forceps can be used for fixation, so as not to cause an adverse effects for other intracavitary operations.

In the surgery, if the outer diameter of the hollow needle 2 is less than 5 mm, the hollow needle 2 enters the cavity through the piercing point, and after the hollow needle 2 is pulled out, because of the self-contracting function of muscles and skins, the piercing point caused by the hollow needle 2 may recover on its own and may no need to be sutured. The wound on the body surface is reduced by using the non-invasive manner. In the present embodiment, the hollow needle includes a tip and a fixed end. The tip of the hollow needle is configured to puncture into the body cavity and fixedly connect with the operating device 1, its outer diameter is 2mm, its inner diameter is 0.8mm, and its length is about 10cm. The fixed end is configured to be connected with the handle 3, and has an outer diameter of about 10mm and a length of about 10 cm. Thus, the length of the most elongate part of the hollow needle 2 can be minimized, which enables the hollow needle 2 to withstand greater force and reduces the vibration of the tip. Preferably, the middle part of the tip is provided with a frosted surface. The middle part of the tip may include several parts in the longitudinal direction, each of which has a predetermined length and is provided with a frosted surface. Alternatively, the middle part is a frosted rod between the tip and the fixed end, the frosted rod has a predetermined length, and two ends of the frosted rod are connected to the tip and the fixed end respectively. The outer surface of the frosted rod is set to be frosted, and its outer diameter and inner diameter are the same as the outer diameter and inner diameter of the tip respectively, and the frosted rod is coaxial with the tip and the fixing end. The predetermined length may be to 1 cm, or the predetermined length can be designed according to different surgeries or different patients. After the hollow needle enters the cavity, the frosted surface can increase the friction between the hollow needle and the wall of the body cavity, increase the tightness of the contact between the hollow needle and the wall of the body cavity, and avoid air leakage during the laparoscopic surgery for not affecting the surgery procedures. In order to prevent the tip of the hollow needle 2 from being damaged by the high strength and high hardness connecting part after the hollow needle 2 enters the fixing groove 121, a rubber protecting block 14 may be arranged at the end of the fixing groove 121 to protect the tip of the hollow needle 2. The rubber block 14 is provided with a hole to facilitate the pull line to pass through. After the operation is finished, the hollow needle 2 is pulled out from the body, there is no scar at the piercing point, and no suturing is needed, so the non-invasive operation is achieved and the operation convenience is improved.

The handle 3 includes a bearing 31, a movable grip 32 and a fixed grip 33. The fixed grip 33 is provided with a mounting hole for fixing the fixed end of the hollow needle 2. When the pull line 124 enters the hollow needle 2 through the tip of the hollow needle 2 and extends out the fixed end of the hollow needle 2, the pull line 124 is twined around the bearing in a predetermined direction and connected with a top end of the movable grip 32. Thus, the surgeon can control the pull line 124 to move within the hollow needle 2 by clenching or loosening the movable grip 32, which drives the retractor clamp to open or close and realizes the purpose of controlling the operating device 1 with the handle 3.

During the surgery, the surgeon makes an incision B on the patient's body surface A according to needs, and then inserts a trocar C corresponding to the size of the incision B to the incision B to form a passage for entry or exit of the surgical instruments. Then, the surgeon places the operating device 1 through the trocar C into the body cavity D. The surgeon makes the handle 3 and the hollow needle 2 fixedly connected, and pierces the hollow needle into the body cavity through a selected piercing point on the body surface A. The hollow needle 2 is fixedly connected with the connecting part 12 of the operating device 1, the hook needle 4 enters the body cavity through the hollow needle 2 at the end of the handle 3, and the pull line 124 is pulled out of the body cavity by the hook needle 4 and is fixedly connected with the handle 3. By clenching the handle 3, the surgeon can control the pull line 124 to move in the connecting part 12, and thus can control the retractor clamp to clamp the organ that needs to be exposed. By controlling the length of the hollow needle 2 inside the body, the organs can be exposed accurately. If necessary, multiple operating devices 1 can be placed in the body cavity to clamp different parts of the tissue so as to better expose the operation part. At the end of the surgery, the retractor clamp is opened by adjusting the length of the pull line 124, and then the pull line 124 is disconnected from the handle 3, so that the hollow needle 2 is separated from the connecting part 12 of the operating device 1. The hollow needle 2 is pulled out from the body, and the operating devices 1 can be taken out of the body cavity through the trocar C. At last, the incision is sutured to finish the operation. In another implementation, the hollow needle 2 is directly cut off, and then the operating device 1 can be taken out of the body though the incision. The intracavitary surgery system of the present embodiment is a non-invasive surgery system and can reduce the injury to the body of the patient. Furthermore, with the intracavitary surgery system of the present embodiment, the pull force and pull direction can be more conveniently adjusted outside the body.

### Second embodiment:

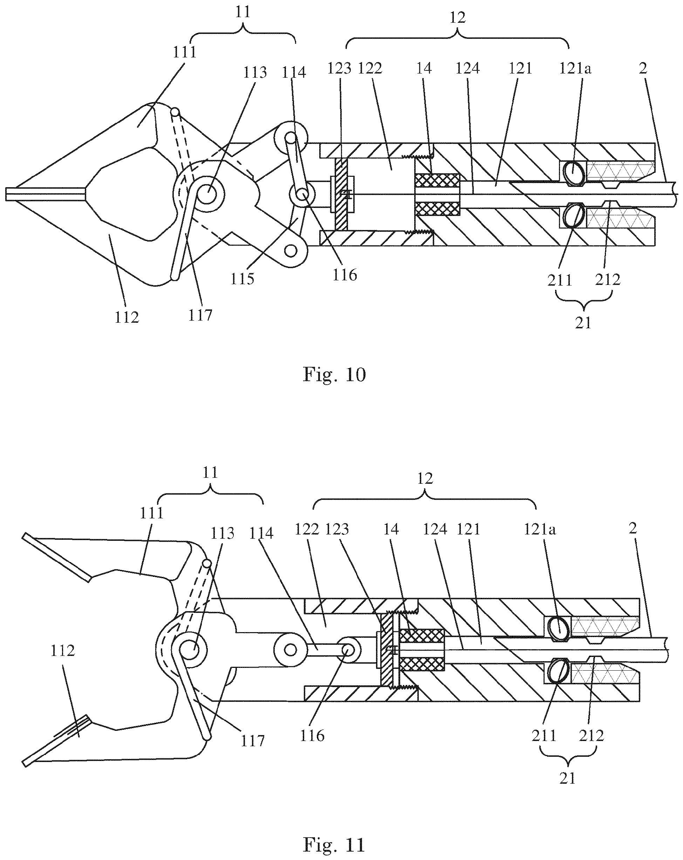

FIG. 10 and FIG. 11 are schematic diagrams of an intracavitary surgery system according to the second embodiment of the present disclosure. In this embodiment of the present disclosure, the connecting rod 2 is a hollow needle. The intracavitary surgery system includes an operating device 1, a connecting rod 2 and a handle 3. In this embodiment of the present disclosure, the operating device 1 is arranged in the thoracic or abdominal cavity for performing the corresponding operation on a lesion of the patient. During the intracavitary surgery, the surgeon makes an incision on the patient's body surface according to needs for forming a passage for entry or exit of the surgical instruments, and then places the operating device 1 through the incision into the body cavity. The hollow needle 2 is fixedly connected with the handle 3. The tip of the hollow needle 2 is pierced into the body cavity through the body surface and is fixedly connected with the operating device 1. The surgeon can control the operating device 1 through the handle 3 outside the body and perform the corresponding operation, which can help avoid the mutual interference between multiple surgical instruments which enter the body cavity through the same incision during surgery, where the mutual interference affects the flexibility of surgical operation such as anatomy or separation, and also can reduce the compression and injury around the incision. If necessary, multiple operating devices 1 can be placed in the body cavity to clamp different parts of the tissue so as to better expose the operation part.

In this embodiment of the present disclosure, the structure of the intracavitary surgery system is basically the same as the structure of the intracavitary surgery system of the first embodiment of the present disclosure. The difference between the two is, when the operating part 11 is a retractor clamp with a first spring 117, the reset spring may be not provided in the connecting part 12 according to needs. The retractor clamp may include a first clamping arm 111 and a second clamping arm 112. The first clamping arm 111 and the second clamping arm 112 may be rotatably connected through a first hinge shaft 113. The first spring 117 is disposed on the first hinge shaft 113. Two ends of the first spring 117 extend and are fixedly connected with the first clamping arm 111 and the second clamping arm 112 respectively. Further, the retractor clamp may be also connected with the connecting part 12 through the first hinge shaft 113. The inner surface of the first clamping arm 111 and the inner surface of the second clamping arm 112 may be provided with antiskid strips to prevent the tissues or organs from slipping during the closing of the retractor clamp. Each of the tail of the first clamping arm 111 and the tail of the second clamping arm 112 is provided with a mounting hole, and the two mounting holes are fixed with the connecting rod 114 and the connecting rod 115 respectively. The other ends of the connecting rod 114 and the connecting rod 115 are rotatably connected through a second hinge shaft 116. In the natural state, there is an included angle between the connecting rod 114 and the connecting rod 115, and the retractor clamp is close. When the slider 123 is pulled by the pull line 124 and moves to the fixing groove 121, the included angle between the connecting rod 114 and the connecting rod 115 decreases gradually, which drives the first clamping arm 111 and the second clamping arm 112 to rotate relatively, and the retractor clamp is in the open state. The retractor clamp is moved to the target tissue or organ, the handle is released, and the retractor clamp returns to the natural state and is in the close state because of the elasticity of the first spring 117 so that the target tissue or organ is clamped. Finally, the clamped tissue or organ is moved as needed to provide a good surgical field of view. The structures of the connecting part 12, the hollow needle 2, the hook needle 4 and the handle 3 in this embodiment are similar to those in the first embodiment, so they will not be repeated here.

### Third embodiment:

In this embodiment of the present disclosure, the intracavitary surgery system includes an operating device 1, a connecting rod 2 and a handle 3. In this embodiment of the present disclosure, the connecting rod 2 is a hollow needle. The operating device 1 is arranged in the thoracic or abdominal cavity for performing the corresponding operation on a lesion of the patient. During the intracavitary surgery, the surgeon makes an incision on the patient's body surface according to needs for forming a passage for entry or exit of the surgical instruments, and then places the operating device 1 through the incision into the body cavity. The hollow needle 2 is fixedly connected with the handle 3. The tip of the hollow needle 2 is pierced into the body cavity through the body surface and is fixedly connected with the operating device 1. The surgeon can control the operating device 1 through the handle 3 outside the body and perform the corresponding operation, which can help avoid the mutual interference between multiple surgical instruments which enter the body cavity through the same incision during surgery, where the mutual interference affects the flexibility of surgical operation such as anatomy or separation, and also can reduce the compression and injury around the incision. If necessary, multiple operating devices 1 can be placed in the body cavity to clamp different parts of the tissue so as to better expose the operation part.

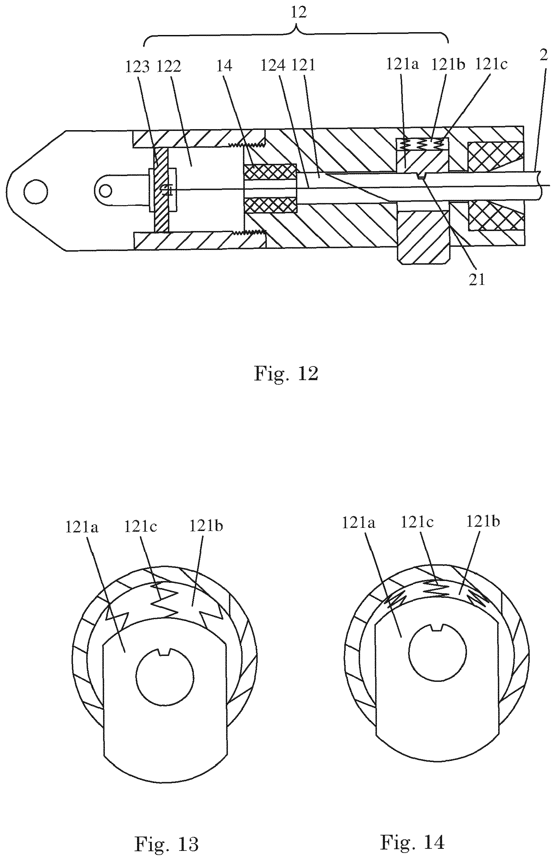

The operating device 1 includes an operating part 11 and a connecting part 12. The operating part 11 may be a retractor clamp, a pair of medical scissors or other surgical instrument suitable for an endoscopic surgery. Referring to FIG. 12 to FIG. 14, The connecting part 12 includes a fixing groove 121, a sliding groove 122, a slider 123 and a pull line 124. A first limiting part 121a for fixing the connecting rod 2 is arranged in the fixing groove 121. The fixing groove 121 is provided with a limiting groove 121b in a longitudinal direction, which is used for accommodating the first limiting part 121a. The tip of the hollow needle 2 is provided with a second limiting part 21 which is matched with the first limiting part 121a. When the tip of the hollow needle 2 enters the fixing groove 121, the hollow needle 2 and the connecting part 12 are fixedly connected through the first limiting part 121a and the second limiting part 21. The connecting part 12 can be made of materials with high strength and hardness such as alloy steel, which can improve the strength of the operating device 1, and can also be used for making small size structures to meet usage requirements. The other structures of hollow needle 2 are similar to those in the first embodiment and will not be described here.

In this embodiment of the present disclosure, the first limiting part 121a may be set to a movable buckle. The first limiting part 121ais fixedly connected to the fixing groove 121 through an elastic element 121c, and moves in the limiting groove according to the elasticity of the elastic element 121c. The movable buckle may be provided with a through-hole, and a fixing buckle is disposed in the through-hole for fixing the hollow needle 2. Corresponding to the first limiting part 121a is a movable buckle, the second limiting part 2lisa clamping groove. In the first state, the through-hole of the first limiting part 121a is coaxial with the fixing groove 121 so that the hollow needle 2 can pass through the through-hole. In the second state, the fixing buckle in the through-hole is mates with the clamping grooves to fix the hollow needle 2. As shown in FIG. 13, when the first limiting part 121a in the fixing groove 121 is in the second state, the center axis of the through-hole of the first limiting part 121a is parallel to the center axis of the fixing groove 121 so that the hollow needle 2 cannot pass through the through-hole. As shown in FIG. 14, when the first limiting part 121a in the fixing groove 121 is in the first state, the center axis of the through-hole of the first limiting part 121a approximately coincides with the center axis of the fixing groove 121 so that the hollow needle 2 can pass through the through-hole.

In this embodiment of the present disclosure, the structures of the operating part 11, the handle 3 the hook needle 4 and the sliding groove 122, the slider 123 and the pull line 124 of the connecting part in this embodiment are similar to those in the first embodiment or the second embodiment, so they will not be repeated here.

### Fourth embodiment:

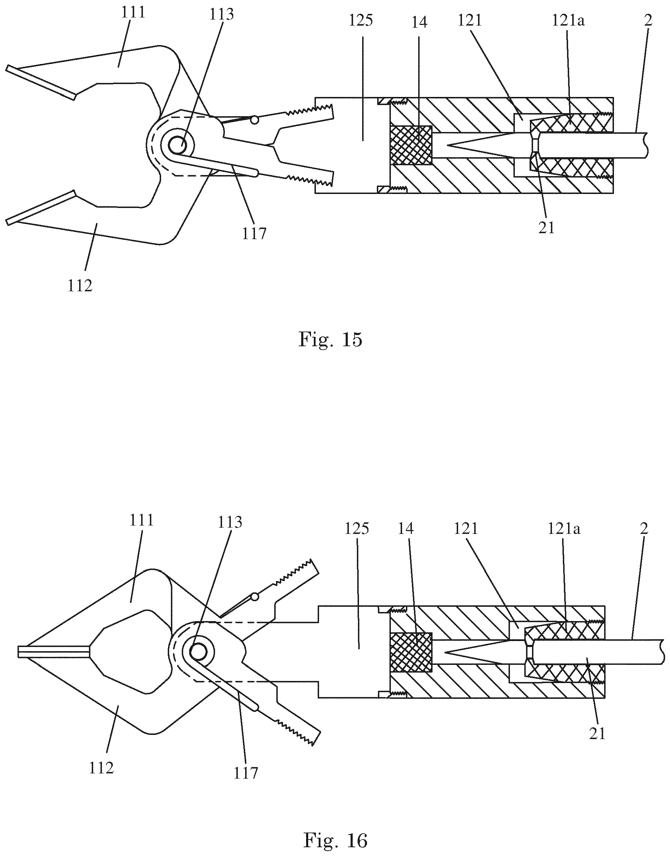

FIG. 15 and FIG. 16 are schematic diagrams of an intracavitary surgery system according to this embodiment of the present disclosure. The intracavitary surgery system includes an operating device 1, a connecting rod 2 and a handle 3. In this embodiment of the present disclosure, the connecting rod 2 may be a solid needle or a hollow needle. The operating device 1 is arranged in the thoracic or abdominal cavity for performing the corresponding operation on a lesion of the patient. During the intracavitary surgery, the surgeon makes an incision on the patient's body surface according to needs for forming a passage for entry or exit of the surgical instruments, and then places the operating device 1 through the incision into the body cavity. The connecting rod 2 is fixedly connected with the handle 3. The tip of the connecting rod 2 is pierced into the body cavity through the body surface and is fixedly connected with the operating device 1. The surgeon can control the operating device 1 through the handle 3 outside the body and perform the corresponding operation, which can help avoid the mutual interference between multiple surgical instruments which enter the body cavity through the same incision during surgery, where the mutual interference affects the flexibility of surgical operation such as anatomy or separation, and also can reduce the compression and injury around the incision.

The operating device 1 includes an operating part 11 and a connecting part 12. In this embodiment of the present disclosure, the operating part His a retractor clamp with a spring. The retractor clamp may include a first clamping arm 111 and a second clamping arm 112. The first clamping arm 111 and the second clamping arm 112 may be rotatably connected through a first hinge shaft 113. The first spring 117 is disposed on the first hinge shaft 113. Two ends of the first spring 117 extend and are fixedly connected with the first clamping arm 111 and the second clamping arm 112 respectively. Due to the elastic force of the first spring 117, the retractor clamp closes in the natural state (no external force is applied). The inner surface of the first clamping arm 111 and the inner surface of the second clamping arm 112 may be provided with antiskid strips to prevent the tissues or organs from slipping during the closing of the retractor clamp.

Referring to FIG. 15 and FIG. 16, the connecting part 12 may include a fixing groove 121 and a connecting piece 125. A first limiting part 121a for fixing the connecting rod 2 is arranged in the fixing groove 121. The tip of the connecting rod 2 is provided with a second limiting part 21 which is matched with the first limiting part 121a. When the tip of the connecting rod 2 enters the fixing groove 121, the connecting rod 2 and the connecting part 12 are fixedly connected through the first limiting part 121a and the second limiting part 21. The connecting part 12 can be made of materials with high strength and hardness such as alloy steel, which can improve the strength of the operating device 1, and can also be used for making small size structures to meet usage requirements.

In this embodiment of the present disclosure, the first limiting part 121a may be set to an elastic jaw, which is embedded in an end of the fixing groove 121. The second limiting part 21 may be a clamping groove. When the connecting rod 2 enters the body cavity through the piercing point, the elastic jaw moves along the tip of the connecting rod 2 based on its elasticity. When the elastic jaw reaching the clamping grooves, the accommodating space becomes larger, so the elastic jaw return to the natural state and be fixedly connected with the clamping groove.

In another implementation, the first limiting part 121a and the second limiting part 21 may also be set to other structures, which are the same as the other first limiting part 121a and the second limiting part 21 in the first embodiment, respectively, and will not be repeated here.

The connecting piece 125 is arranged on the side of the connecting part 12 opposite to the sliding groove 121 and extends along the sliding groove 121. The connecting piece 125 is further provided with a connecting hole. The first hinge shaft 113 extends through the connecting hole and connects the retractor clamp with the connecting part 12.

In the surgery, if the connecting rod 2 is a hollow needle, the outer diameter of the hollow needle is generally less than 5 mm. The hollow needle includes a tip and a fixed end. The tip of the hollow needle is configured to puncture into the body cavity and fixedly connect with the operating device 1, its outer diameter is 2mm, its inner diameter is 0.8mm, and its length is about 10 cm. The fixed end is configured to be connected with the handle 3, and has an outer diameter of about 10mm and a length of about 10 cm. Thus, the length of the most elongate part of the hollow needle 2 can be minimized, which enables the hollow needle 2 to withstand greater force and reduces the vibration of the tip. Preferably, the middle part of the tip is provided with a frosted surface. The middle part of the tip may include several parts in the longitudinal direction, each of which has a predetermined length and is provided with a frosted surface. Alternatively, the middle part is a frosted rod between the tip and the fixed end, the frosted rod has a predetermined length, and two ends of the frosted rod are connected to the tip and the fixed end respectively. The outer surface of the frosted rod is set to be frosted, and its outer diameter and inner diameter are the same as the outer diameter and inner diameter of the tip respectively, and the frosted rod is coaxial with the tip and the fixing end. The predetermined length may be to 1 cm, or the predetermined length can be designed according to different surgeries or different patients. After the hollow needle enters the cavity, the frosted surface can increase the friction between the hollow needle and the wall of the body cavity, increase the tightness of the contact between the hollow needle and the wall of the body cavity, and avoid air leakage during the laparoscopic surgery for not affecting the surgery procedures. In order to prevent the tip of the hollow needle 2 from being damaged by the high strength and high hardness connecting part after the hollow needle 2 enters the fixing groove 121, a rubber protecting block 14 may be arranged at the end of the fixing groove 121 to protect the tip of the hollow needle 2. If the connecting rod 2 is a solid needle, the diameter of the solid needle is less than 5 mm. The structure of the solid needle is basically similar to that of the hollow needle except that it has no inner hole.

During the surgery, the operating device 1 is placed into the body cavity through an incision. The connecting rod 2 fixed with the handle 3 is pierced into the body cavity through a selected piercing point on the body surface. The connecting rod 2 is fixedly connected with the connecting part 12 of the operating device 1. The retractor clamp is controlled by handle 3 to move to the organ to be exposed, and then a separation forceps is sent into the cavity through the incision. Under the guidance of the endoscope, the separation forceps control the retractor clamp to hold the organs that need to be exposed. Then, the length of the connecting rod 2 in the body is adjusted through the handle 3 to achieve the precise exposing of the organ. After the surgery is complete, the connecting rod 2 is cut off, the operating device 1 is taken out of the body through the incision. The intracavitary surgery system in the present embodiment can reduce the damage to the patients and can facilitate the control of the retracting force and the retracting direction from outside of the body.

The intracavitary surgery system of embodiments of the present embodiment includes an operating device, a connecting rod and a handle. The operating device includes an operating part and a connecting part. During the surgery, the operating device can be placed in the body cavity through an incision, and the tip of the connecting rod enters the body cavity through the piercing point and is fixedly connected with the connecting part of the operating device. The other end, outside the body, of the connecting rod is connected with the handle. Then, the operating part can be controlled and adjusted through the handle outside the body to achieve the corresponding surgery procedures. The present disclosure provides a non-invasive way to reduce the injury to the patient's body, makes the surgical procedures outside the body more convenient, and improves the success rate of the operation.

The above description is only preferred embodiments of the present invention and is not intended to limit the present invention. For those skilled in the art, the present invention may have various modifications and changes. Any modification, equivalent replacement, improvement, etc. made within the spirit and principle of the present invention shall be included in the protection scope of the present invention.

## Claims

1. An intracavitary surgery system, comprising:
a needle type connecting rod;
an operating device comprising an operating part (11) and a connecting part (12); and
a handle (3) for fixing the connecting rod (2);
wherein the connecting part is configured to be fixedly connected with the tip of the connecting rod (2) in the body cavity.

2. The intracavitary surgery system according to claim 1, wherein the connecting part (12) comprises:
a fixing groove (121) for accommodating the tip of the connecting rod (2);
wherein a first limiting part (121a) for fixing the connecting rod (2) is arranged in the fixing groove (121).

3. The intracavitary surgery system according to claim 2, wherein the tip of the connecting rod (2) is provided with a second limiting part (21) matched with the first limiting part (121a).

4. The intracavitary surgery system according to claim 3, wherein the first limiting part (121a) is a buckle, and the second limiting part (21) is a clamping groove.

5. The intracavitary surgery system according to claim 3, wherein the tip of the connecting rod (2) is configured to be fixedly connected with fixing groove (121) by threads.

6. The intracavitary surgery system according to claim 3, wherein the first limiting part (121a) comprises at least one elastic ring.

7. The intracavitary surgery system according to claim 6, wherein the second limiting part (21) is provided with a clamping groove (211) and a guide groove (212), wherein the connecting rod (2) is fixed with the elastic ring by the clamping groove (211), and is separated from the elastic ring by the guiding groove (212).

8. The intracavitary surgery system according to claim 3, wherein the first limiting part (121a) is provided with a through-hole, wherein the first limiting part (121a) is fixedly connected with the fixing groove (121) by an elastic element (121c).

9. The intracavitary surgery system according to claim 8, wherein the through-hole is provided with a fixing buckle for fixing the connecting rod (2).

10. The intracavitary surgery system according to claim 9, wherein the second limiting part (21) is a clamping groove; in a first state, the through-hole of the first limiting part (121a) is coaxially disposed with the fixing groove (121) to make the connecting rod (2) go through the through-hole; and in a second state, the buckle in the through-hole is engaged with the clamping groove to fix the connecting rod (2).

11. The intracavitary surgery system according to claim 3, wherein the first limiting part (121a) is an elastic jaw.

12. The intracavitary surgery system according to claim 11, wherein the second limiting part (21) is a clamping groove, wherein the clamping groove is configured to be fixedly connected with the jaw to fix the connecting rod (2).

13. The intracavitary surgery system according to claim 2, wherein a rubber block (14) may be arranged at one end of the fixing groove (121) close to the operating part (11) to protect the tip of the connecting rod (2).

14. The intracavitary surgery system according to claim 2, wherein the connecting part (12) further comprises:
a sliding groove (122) arranged opposite to the fixing groove (121);
a slider (123) arranged in the sliding groove (122), wherein the slider (123) and the operating part (11) are connected; and
a pull line (124) with one end fixedly connected with the slider (123) to drive the slider (123) to move.

15. The intracavitary surgery system according to claim 14, further comprising:
a hook needle, wherein an end of the hook needle is provided with a groove or a hook.

16. The intracavitary surgery system according to claim 2, wherein the connecting part (12) further comprises:
a connecting piece (125) extends along a side of the sliding groove (121).

17. The intracavitary surgery system according to claim 16, wherein the operating part (11) is a retractor clamp, wherein the retractor clamp is connected with the connecting piece (125).

18. The intracavitary surgery system according to claim 17, wherein the retractor clamp comprises a first clamping arm (111) and a second clamping arm (112), wherein the first clamping arm (111) and the second clamping arm (112) are rotatably connected by a first hinge shaft (113).

19. The intracavitary surgery system according to claim 17, wherein the first hinge shaft (113) is provided with a first spring (117), two ends of the first spring (117) extend and are fixedly connected with the first clamping arm (111) and the second clamping arm (112) respectively.

20. The intracavitary surgery system according to claim 1, wherein the diameter of the connecting rod (2) is less than 5mm.

21. The intracavitary surgery system according to claim 20, wherein the surface of the middle part of the connecting rod (2) is provided with a frosted surface.
